# EUROPÄISCHE PATENTSCHRIFT

(11) **EP 1 556 488 B1**
(45) Veröffentlichungstag und Bekanntmachung des Hinweises auf die Patenterteilung: **20.08.2008**
(21) Anmeldenummer: 03758057.8
(22) Anmeldetag: 24.10.2003
(51) Int. Cl.: C12N 15/12, C07K 14/47, C07K 16/18, G01N 33/50, G01N 33/53, A61K 38/17, A61P 3/04, A61P 3/10, A61P 5/18, A61P 13/12, A61P 17/00, A61P 19/00, A61P 29/00, A61P 37/00

(54) **HUMANES CHONDROOSTEOMODULIN (TIG2), HERSTELLUNG UND VERWENDUNG ZUR BEHANDLUNG ODER DIAGNOSE VON KNOCHEN- UND KNORPELERKRANKUNGEN, FETTSUCHT SOWIE ENTZÜNDLICHEN ERKRANKUNGEN UND HAUTERKRANKUNGEN**
HF-CHONDROOSTEOMODULIN, PRODUCTION, AND USE FOR THE TREATMENT OR DIAGNOSIS OF BONE DISEASES, CARTILAGE DISEASES, OBESITY, INFLAMMATORY DISEASES, AND SKIN DISEASES
HF-CHONDROOSTEOMODULINE, PRODUCTION ET UTILISATION POUR REALISER LE TRAITEMENT OU LE DIAGNOSTIC DE MALADIES DES OS OU DES CARTILAGES, DE L'ADIPOSE, DE MALADIES INFLAMMATOIRES ET DE MALADIES DE LA PEAU

(30) Priorität: 31.10.2002 DE 10251205
(43) Veröffentlichungstag der Anmeldung: 27.07.2005
(73) Patentinhaber: IPF Pharmaceuticals GmbH, 30625 Hannover (DE)
(72) Erfinder: MEDER, Wolfgang, 30625 Hannover (DE); WENDLAND, Martin, 30625 Hannover (DE); JOHN, Harald, 30625 Hannover (DE); RICHTER, Rudolf, 30625 Hannover (DE); MEYER, Markus, 30625 Hannover (DE); FORSSMANN, Wolf-Georg, 30625 Hannover (DE)
(74) Vertreter: Meyers, Hans-Wilhelm
(86) Internationale Anmeldenummer: PCT/EP2003/011799
(87) Internationale Veröffentlichungsnummer: WO 2004/039978

(56) Entgegenhaltungen:
- WO-A-03/006996
- NAGPAL, S. ET AL.: "Tazarotene-induced Gene 2 (TIG2), a Novel Retinoid-Responsive Gene in Skin" JOURNAL OF INVESTIGATIVE DERMATOLOGY, Bd. 109, Nr. 1, Juli 1997 (1997-07), Seiten 91-95, XP009018662 in der Anmeldung erwähnt -& DATABASE EMBL, HEIDELBERG, BRD [Online] 30. Mai 2000 (2000-05-30), NAGPAL, S. ET AL.: "Retinoic acid receptor responder protein 2 precursor (Tazarotene-induced gene 2 protein) (RAR-responsive protein TIG2)" XP002273640 Database accession no. Q99969
- ADAMS, A.E. ET AL.: "1,25 Dihydroxyvitamin D3 and Dexamethasone Induce the Cyclooxygenase 1 Gene in Osteoclast-Supporting Stromal Cells" JOURNAL OF CELLULAR BIOCHEMISTRY, Bd. 74, Nr. 4, 15. September 1999 (1999-09-15), Seiten 587-595, XP002273638 in der Anmeldung erwähnt
- SAMSON, M. ET AL.: "ChemR23, a putative chemoattractant receptor, is expressed in monocyte-derived dendritic cells and macrophages and is a coreceptor for SIV and some primary HIV-1 strains" EUROPEAN JOURNAL OF IMMUNOLOGY, Bd. 28, Nr. 5, Mai 1998 (1998-05), Seiten 1689-1700, XP001155279 in der Anmeldung erwähnt
- METHNER, A. ET AL.: "A Novel G Protein-coupled Receptor with Homology to Neuropeptide and Chemoattractant Receptors Expressed during Bone Development" BIOCHEMICAL AND BIOPHYSICAL RESEARCH COMMUNICATIONS, Bd. 233, Nr. 2, 17. April 1997 (1997-04-17), Seiten 336-342, XP002204803 in der Anmeldung erwähnt
- SCHULZ-KNAPPE, P. ET AL.: "Peptide bank generated by large-scale preparation of circulating human peptides" JOURNAL OF CHROMATOGRAPHY A, Bd. 776, Nr. 1, 25. Juli 1997 (1997-07-25), Seiten 125-132, XP004125547
- WITTAMER, V. ET AL.: "Specific Recruitment of Antigen-presenting Cells by Chemerin, a Novel Processed Ligand from Human Inflammatory Fluids" JOURNAL OF EXPERIMENTAL MEDICINE, Bd. 198, Nr. 7, 6. Oktober 2003 (2003-10-06), Seiten 977-985, XP002273639
- MEDER, W. ET AL.: "Characterization of human circulating TIG2 as a ligand for the orphan receptor ChemR23" FEBS LETTERS, Bd. 555, Nr. 3, 18. Dezember 2003 (2003-12-18), Seiten 495-499, XP004481038

## Beschreibung

Die Erfindung betrifft das Polypeptid HF-Chondroosteomodulin (COM) und seine Derivate, sowie Verfahren zur Herstellung und Gewinnung in reiner oder partiell aufgereinigter Form aus Körperflüssigkeiten und Geweben oder durch chemische oder biotechnologische Synthese.

Der Erfindung liegt die Aufgabe zugrunde, weitere oder verbesserte Wirkstoffe zur Behandlung oder Diagnose von Knochen- und Knorpelerkrankungen, Fettsucht sowie inflammatorischer Erkrankungen und Hauterkrankungen bereitzustellen.

### Figuren:

### Figur 1

Spezifische Aktivierung von GORI-28 Zellen (Klon C1-6 und C1-5) durch Fraktionen 22-25 des pH Pools 7. Die Kontrollzellen (control) exprimieren den GORI-28 nicht. Unspezifische Aktivität ist in den Fraktionen 15-16 zu erkennen.

### Figur 2

Schritt 3 der chromatographischen Reinigung von COM über eine Bakerbond RP-C18 Säule.

### Figur 3

Schritt 7 der chromatographischen Reinigung von COM über eine Poly Hydroxyethyl HILIC Säule.

### Figur 4

Dosis-Wirkungs-Korrelation. Aufgetragen ist die Veränderung der Fluoreszenz gegen das eingesetzte Volumen von Fraktion 30 des 8. Aufreinigungsschrittes (siehe Beispiel 4).

### Figur 5

Dosis-Wirkungs-Kurve. Aufgetragen ist die Veränderung der Fluoreszenz gegen das eingesetzte Volumen von Fraktion 30 des 8. Aufreinigungsschrittes (siehe Beispiel 4) im halblogarithmischen Maßstab.

### Figur 6

Aktivierung von GORI-28 Zellen mit rekombinantem TIG2 aus konditioniertem Medium von überexprimierenden CHO Zellen gereinigt über eine Source RPC15 (10 x 250 mm)

### Figur 7

Aktivierung von GORI-28 Zellen mit rekombinanten COM gereinigt aus Zellüberstand von einem überexprimierenden Hefeklon.

### Figur 8

Aktivierung von osteogenen Zellen (MG-63) und dendritischen Zellen (DC) mit COM gereinigt aus Hämofiltrat. Als Positivkontrolle bzw. Negativkontrolle sind GORI-28 exprimierende bzw. nicht-exprimierende CHO-Zellen gezeigt.

### Figur 9

Expression von GORI-28 und TIG2 in verschiedenen Zelltypen

### Figur 10

Expression von GORI-28 und TIG2 in Hautproben von Patienten mit Hauterkrankungen

Die der Erfindung zugrunde liegende Aufgabe wird gelöst durch COM mit der Aminosäuresequenz SEQ ID Nr. 1 
1 ELTEAQRRGL QVALEEFHKH PPVQWAFQET SVESAVDTPF PAGIFVRLEF
51 KLQQTSCRKR DWKKPECKVR PNGRKRKCLA CIKLGSEDKV LGRLVHCPIE
101 TQVLREAEEH QETQCLRVQR AGEDPHSFYF PGQF
 sowie amidierte, acetylierte, phosphorylierte und glycosylierte Derivate oder mit einem Pyroglutamat am N-Terminus.

Gegenstand der Erfindung sind auch Derivate von COM, wobei die Aminosäuresequenz von COM durch Aminosäureaustausche, -insertionen oder -deletionen verändert ist, mit den Massgaben, dass
- die Derivate eine Länge von 129 bis 140 Aminosäuren aufweisen,
- die Derivate zu COM zu mehr als 80%, vorzugsweise mehr als 90%, insbesondere mehr als 95% Sequenzidentität aufweisen,
- die Derivate im funktionellen Test mit dem FLIPR-System den Rezeptor GORI-28 aktivieren, so dass eine Rezeptoraktivität gemessen wird, die mindestens 50% der unter gleichen Testbedingungen von COM ausgelösten Rezeptoraktivität beträgt, vorzugsweise jedoch größer ist als diese Aktivität, und besonders bevorzugt diese um mindestens 20% übertrifft.

Die der Erfindung zugrunde liegende Aufgabe wird außerdem gelöst durch die weiteren Ausführungsformen der Erfindung gemäß den Ansprüchen 3 bis 12.
COM besitzt die Fähigkeit, Knochenzellen (Osteoblasten und Osteoklasten) und Knorpelzellen, sowie Fett- Immun- und Hautzellen in ihrer Funktion zu beeinflussen. Der Stoff kann aus der Körperflüssigkeit menschliches Blutfiltrat (Hämofiltrat, HF) gewonnen werden. Der Stoff ist als COM bezeichnet und kann zum Zwecke der medizinischen und gewerblichen Verwendung als Medikament zur Behandlung oder Diagnose von Knochen-und Knorpelerkrankungen, sowie Fettsucht, Diabetis Typ 2, Krebserkrankungen, Tumor Metastasen, inflammatorischen Erkrankungen, Autoimmunerkrankungen, vererbten oder erworbenen Immundefizienzen, Gewebeabstoßung, Hauterkrankungen wie Psoriasis, Ekzeme, Akne oder trophischen Hauterkrankungen, inflammatorischen Infektionen, viralen, bakteriellen oder parasitischen Infektionen, weiblicher Infertilität, Ovar- und Uterustumoren benutzt werden.

Zur Analyse von Stoffen aus Peptidbänken wurde ein Bioassay entwickelt, der an transfizierten CHO-Zellen eine Aktivierung der Signaltransduktion zeigt. Überraschenderweise wurden im Blutfiltrat aktivierende Substanzen gefunden, die in Zellkulturen von GORI-28-Rezeptoren transfizierter Zellen, auch bekannt als ChemR23 (Genbank Accession No. Y14838) oder DEZ (Genbank Accession No. U79527), die intrazelluläre Ca⁺⁺-Aktivierung beeinflussen. Dieser Rezeptor ist funktionell auf osteogenen Zellen, Adipozyten, Hautzellen sowie auf Immunzellen aktiv. Die Beeinflussung wurde u.a. gemessen aufgrund der Stimulierung von intrazellulärer Ca⁺⁺-Aktivierung von Zellen, die auf der Membranoberfläche den GORI-28-Rezeptor tragen. Mit Hilfe dieses Testes konnte aus HF-Peptidbank (menschliches Blutfiltrat) überraschenderweise eine Substanz identifiziert werden, die charakteristische Elutionsprofile in den Chromatographieaufreinigungen zeigt. Die Substanz wurde isoliert und durch massenspektrometrische, einschlägige Analysen als molekulare Einheit identifiziert. Es handelt sich um eine zirkulierende Form des bereits aus Klonierung gefundenen TIG2 (Tazarotene-induced responder protein 2; Nagpal S. et al. (1997) J. Invest. Dermatol., 109: 91-95) (Genbank Accession No. U77594).

Die cDNA für den GORI-28 wurde über PCR aus genomischer DNA kloniert und in einen eukaryotischen Expressionsvektor subkloniert. Es wurden stabil transfizierte CHO-Zellinien erzeugt, die den GORI-28 überexprimieren und anschließend in einem funktionellen Screening Assay eingesetzt. Dabei wurden GORI-28 exprimierende Zellen mit Fraktionen aus HF stimuliert und die Rezeptoraktivierung über die transiente Erhöhung des Second Messenger Ca2+ verfolgt. Liganden sind bisher für den Rezeptor GORI-28 nicht beschrieben worden und der Rezeptor ist deshalb als Orphan Rezeptor klassifiziert worden.

Der GORI-28 Rezeptor wird auf sich entwickelnden Knochen- und Knorpelzellen, sowie auf dendritischen Zellen, in Lymphknoten, Milz, Plazenta, Uterus, Lunge, Aorta und in der adulten Nebenschilddrüse exprimiert (Samson et al., 1998, Eur. J. Immunol. 28:1689-1700, Methner et al., 1997, Biochem Biophys Res Commun 233:336-342).

TIG2 wird im Pankreas, Leber, Adipocyten, Nebenniere, Lunge, Ovar, Uterus, Hypophyse, Epidermiszellen und Osteoklasten unterstützenden Stromazellen exprimiert (Nagpal S. et al., 1997, Adams et al., 1999, J. Cell. Biochem. 74:587-595). Zudem wurde beobachtet, dass TIG2 im geschädigten Gewebe von Psioriasis-Patienten im Vergleich zu nicht geschädigtem Gewebe in verminderter Menge exprimiert wird. Nach Behandlung von geschädigtem Gewebe mit Tazaroten wird die TIG2 Expression induziert (Nagpal S. et al., 1997).

Aus diesen und in den Beispielen aufgeführten Daten leiten sich physiologische Bedeutung von COM und GORI-28 bei der Knochenentwicklung, des Energiestoffwechsels, der Physiologie und Erhaltung der Haut und von inflammatorischen Prozessen ab.

COM wird als körpereigenes zirkulierendes Peptid für eine Anwendung als Arzneimittel besser geeignet sein, als andere Derivate des TIG2 Gens, da es vom menschlichen Immunsystem nicht als Fremdsubstanz erkannt wird, und vorteilhafterweise nicht mit einer Autoimmunantwort zu rechnen ist. Zudem lässt die spezifische Prozessierung sowohl am N- wie am C-Terminus erwarten, dass dies zur Ausbildung der biologischen Funktion erforderlich ist und zu einer höheren Aktivität führt.

Die vorliegende Erfindung betrifft somit einen neuen osteochondroaktiven Faktor, COM, mit den folgenden molekularen Eigenschaften:
(1) Molekulargewicht des Precursors (aus Gendaten): 18617 Da
(2) Molekulargewicht des gefundenen Proteins COM: 15566 Da
(3) Chromatographisches Verhalten: siehe Beispiel 5
(4) Aminosäuresequenz: gemäß Anspruch 1
(5) pI-Wert: 8.60

### Beispiele

### Beispiel 1: Klonierung des GORI-28/ChemR23 Rezeptors

Der GORI-28 wurde von Samson et al. (1998) als ChemR23 und von Methner et al. (1997) als DEZ beschrieben, wobei mehrere Datenbankeinträge existieren. Die Analyse eines genomischen Klons (Genbank Accession No. NT_009660.4) zeigte, dass die beschriebenen Varianten von DEZ Isoform A (Genbank Accession No. U79526) und Isoform B (Genbank Accession No. U79527) die Produkte von alternativem Splicing darstellen. DEZ Isoform A ist identisch in der Aminosäuresequenz zu ChemR23 und DEZ Isoform B, wobei der N-Terminus von Isoform A um zwei Aminosäuren, Methionin und Arginin, verlängert ist.

Um eine erleichterte Klonierung aus genomischer DNA zu ermöglichen wurde *in silico* ein genomischer Klon für ChemR23/DEZ Isoform B erzeugt, der die Bezeichnung GORI-28 erhielt. Der GORI-28 enthält gegenüber der Sequenz von ChemR23 (Genbank Accession No. Y14838) in der Position 900 ein Guanin, was eine stille Mutation darstellt, und ab Position 1294 eine von der publizierten Sequenz völlig abweichende Sequenz. Die cDNA für GORI-28 wurde mit den Primer 5' TGG TCC CTG TCT TCT CTT GC 3' (GORI28oli1) und 5' TGT CCC TGG GTT GAG AGA GT 3'(GORI28oli2) über PCR aus genomischer DNA amplifiziert. Dabei wurde ein 1186 bp Fragment erhalten, das anschließend in den Expressionsvektor pCI oder andere übliche Expressionsvektoren subkloniert wurde. Die Sequenz wurde über DNA-Sequenzanalyse überprüft und bestätigt.

Die GORI-28 cDNA weist folgende Polynukleotidsequenz SEQ ID Nr. 2 auf:

1 TGGTCCCTGT CTTCTCTTGC AGAGAATGGA GGATGAAGAT TACAACACTT
51 CCATCAGTTA CGGTGATGAA TACCCTGATT ATTTAGACTC CATTGTGGTT
101 TTGGAGGACT TATCCCCCTT GGAAGCCAGG GTGACCAGGA TCTTCCTGGT
151 GGTGGTCTAC AGCATCGTCT GCTTCCTCGG GATTCTGGGC AATGGTCTGG
201 TGATCATCAT TGCCACCTTC AAGATGAAGA AGACAGTGAA CATGGTCTGG
251 TTCCTCAACC TGGCAGTGGC AGATTTCCTG TTCAACGTCT TCCTCCCAAT
301 CCATATCACC TATGCCGCCA TGGACTACCA CTGGGTTTTC GGGACAGCCA
351 TGTGCAAGAT CAGCAACTTC CTTCTCATCC ACAACATGTT CACCAGCGTC
401 TTCCTGCTGA CCATCATCAG CTCTGACCGC TGCATCTCTG TGCTCCTCCC
451 TGTCTGGTCC CAGAACCACC GCAGCGTTCG CCTGGCTTAC ATGGCCTGCA
501 TGGTCATCTG GGTCCTGGCT TTCTTCTTGA GTTCCCCATC TCTCGTCTTC
551 CGGGACACAG CCAACCTGCA TGGGAAAATA TCCTGCTTCA ACAACTTCAG
601 CCTGTCCACA CCTGGGTCTT CCTCGTGGCC CACTCACTCC CAAATGGACC
651 CTGTGGGGTA TAGCCGGCAC ATGGTGGTGA CTGTCACCCG CTTCCTCTGT
701 GGCTTCCTGG TCCCAGTCCT CATCATCACA GCTTGCTACC TCACCATCGT
751 GTGCAAACTG CAGCGCAACC GCCTGGCCAA GACCAAGAAG CCCTTCAAGA
801 TTATTGTGAC CATCATCATT ACCTTCTTCC TCTGCTGGTG CCCCTACCAC
851 ACACTCAACC TCCTAGAGCT CCACCACACT GCCATGCCTG GCTCTGTCTT
901 CAGCCTGGGT TTGCCCCTGG CCACTGCCCT TGCCATTGCC AACAGCTGCA
951 TGAACCCCAT TCTGTATGTT TTCATGGGTC AGGACTTCAA GAAGTTCAAG
1001 GTGGCCCTCT TCTCTCGCCT GGTCAATGCT CTAAGTGAAG ATACAGGCCA
1051 CTCTTCCTAC CCCAGCCATA GAAGCTTTAC CAAGATGTCA TCAATGAATG
1101 AGAGGACTTC TATGAATGAG AGGGAGACCG GCATGCTTTG ATCCTCACTG
1151 TGGAACCCCT CAATGGACTC TCTCAACCCA GGGACA

### Beispiel 2: Erzeugung von GORI-28 überexprimierenden CHO Zellen

Der Expressionsvektor mit der cDNA für GORI-28 wurde mit dem Transfektionsreagenz Effectene oder anderen üblichen Transfektionsreagenzien nach Angaben der Hersteller in CHO Zellen transfiziert, die endogen das G-Protein α16 exprimieren. Stabil transfizierte Zellklone wurden in Gegenwart von Neomycin (G-418) selektioniert und die erhaltenen Zellklone über Northern Blot Analyse auf Expression des GORI-28 untersucht. Zellklone mit unterschiedlich starker Expression (GORI-28 C1-5, C1-6, C1-8) wurden für das Screening mit Peptidfraktionen ausgewählt (s. Beispiel 3)

### Beispiel 3: Funktioneller Test für GORI-28

Mit Hilfe des FLIPR-Systems (Fluorometric Imaging Plate Reader, Molecular Devices) können Veränderungen der intrazellulären Kalziumkonzentration detektiert werden. Nach Rezeptorstimulation wird der intrazelluläre Botenstoff IP3 freigesetzt, der IP3-spezifische Känale des endoplasmatischen Retikulums öffnet und den Ausstrom von Ca²⁺-Ionen ins Cytosol bewirkt. Vor der Messung werden die Zellen mit dem Kalzium-sensitiven Farbstoff Fluo-4 (Molecular Probes) beladen. Ca²⁺-Ionen binden an Fluo-4 und nach Anregung des Fluo-4-Ca²⁺ Komplexes durch einen Argonlaser (488 nm) wird die Emission bei einer Wellenlänge von 540 nm gemessen. Dieses Lichtsignal wird von einer CCD-Kamera detektiert und aufgezeichnet und anschließend mit einem Computerprogramm ausgewertet. Zellen werden in 96-Lochplatten mit 20,000 Zellen/Loch ausgesät und über Nacht inkubiert. Am folgenden Tag werden die Zellen für 40 min in Hepes/HBSS Puffer, pH7.4, 2.5 mM Probenecid mit 2 µM Fluo-4 AM beladen, anschließend gewaschen und mit 100 µl Hepes/HBSS, pH7.4, 2.5 mM Probenecid für 5 min inkubiert. Nach Zugabe von 50 µl Hämofiltratfraktion oder anderer Testsubstrate werden die Veränderungen der intrazellulären Fluoreszenz online aufgezeichnet.

### Beispiel 4: Isolierung von HF-Chondroosteomodulin

Die Isolierung von COM aus 8.000 Liter menschlichem Blutfiltrat wurde nach folgendem Beispiel vorgenommen:

| **Isolierung von HF-Chondroosteomodulin aus Hämofiltrat: Reinigungsstrategie** | |
|---|---|
| **Kollektion von 8.000 L-Batch menschlichen Hämofiltrats** | |
| **Kationen-Austauscher-Chromatographie (Stufenelution)** | **1. Schritt** |
| **RP-Fineline Source C15-Chromatographie (Gradientenelution)** | **2. Schritt** |
| **Bakerbond RP C18-Säulenchromatographie (Gradientenelution)** | **3. Schritt** |
| **RP-Biotek C4-Säulenchromatographie (Gradientenelution)** | **4. Schritt** |
| **RP-Vydac C18-Säulenchromatographie (Gradientenelution)** | **5. Schritt** |
| **RP-Phenomenex C5-Säulenchromatographie (Gradientenelution)** | **6. Schritt** |
| **Poly Hydroxyethyl HILIC-Säulenchromatographie (Gradientenelution)** | **7. Schritt** |
| **RP-Phenomenex C5-Säulenchromatographie (Gradientenelution)** | **8. Schritt** |
| **Hohe Reinheit erreicht** | |

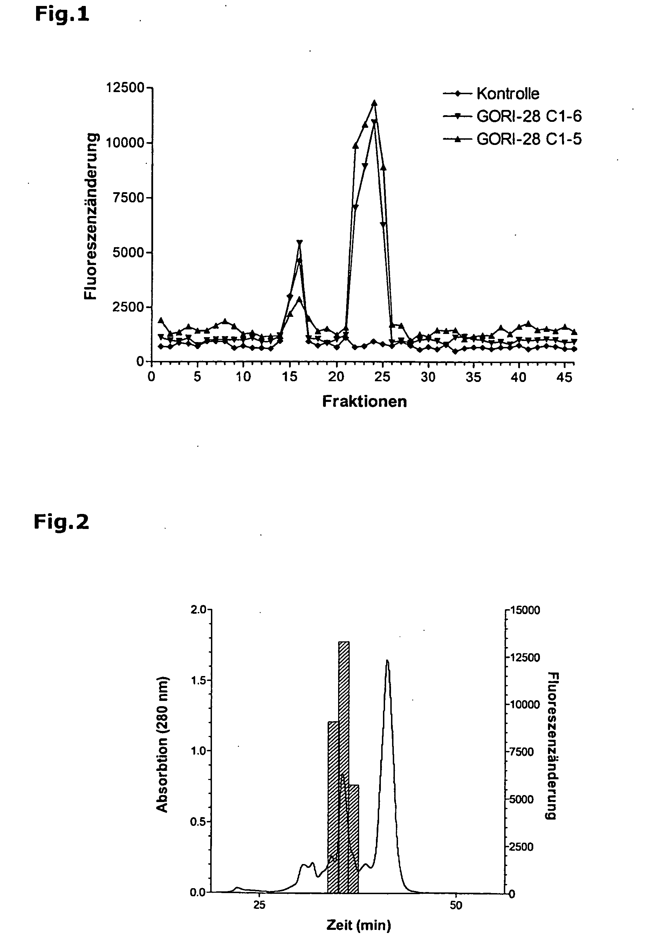

8000 Liter Hämofiltrat wurden über eine Membran mit einer Ausschlußgröße von 50 kD ultrafiltriert, anschließend an eine Kationen-Austauscher-Säule (Fractogel SP 650 (M)) gebunden und danach stufenweise mit 7 Puffern mit aufsteigendem pH-Wert eluiert (pH Pool 1-7, 7 Eluate). In einem zweiten Schritt wurde jedes Eluat über eine Fineline Source RP-C (C15, 10 x 15.5 cm, 300 Å) chromatographiert, so dass für jeden pH Pool etwa 46 Fraktionen erhalten wurden. Für den Biotest wurden lyophilisierte Aliquots in Hepes/HBSS Puffer rekonstituiert und wie in Beispiel 3 beschrieben verwendet. Dabei zeigten Fraktionen 22-25 des pH Pools 7 eine spezifische Aktivität auf GORI-28 Zellen, nicht aber auf einer Kontrollzellinie, die den Rezeptor nicht exprimiert (s. Fig. 1). Etwa 700 mg lyophilisierte Mutterfraktionen der GORI-28-aktivierenden Fraktionen wurden vereinigt und in sechs weiteren Schritten (s. Schema) aufgereinigt. Das zur hohen Reinheit aufgetrennte COM zeigt die chromatographischen, massenspektrometrischen und molekularen Eigenschaften wie in Beispiel 5 gezeigt.

### Beipiel 5: Chromatographische, massenspektrometrische und molekularen Eigenschaften

Aus der chromatographischen Aufreinigung von COM sind beispielhaft die Schritte 3 und 7 in Fig. 1 und Fig. 2 dargestellt, wobei die Fraktionen, in denen die biologische Aktivität gefunden wurde, markiert sind.

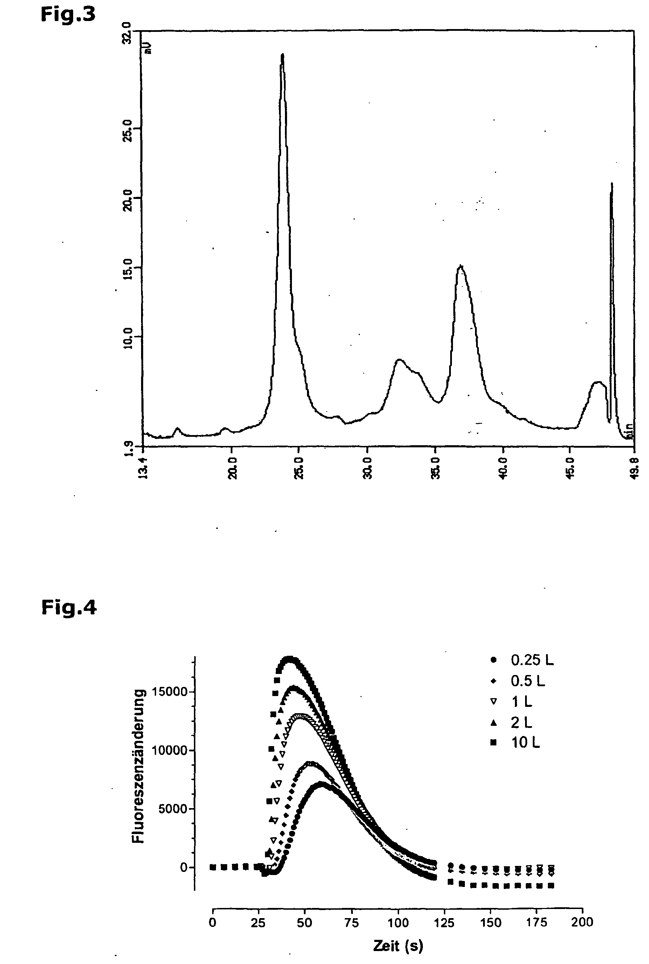

In Fig. 4 ist eine Dosis-Wirkungs-Korrelation von COM dargestellt. Aufgetragen ist die Veränderung der Fluoreszenz gegen das eingesetzte Volumen von Fraktion 30 des 8. Aufreinigungsschrittes (siehe Beispiel 4).
In Fig. 5 ist eine Dosis-Wirkungs-Kurve von COM dargestellt. Aufgetragen ist die Veränderung der Fluoreszenz gegen das eingesetzte Volumen von Fraktion 30 des 8. Aufreinigungsschrittes (siehe Beispiel 4).

Die Reinheit von COM wurde über Kapillarzonenelektrophorese (P/ACE 2000, Beckman) überprüft (nicht dargestellt). Die Bestimmung der molekularen Masse erfolgte über ein Voyager DE PRO Massenspektrometer (PerSpective), wobei eine Masse von 15562 Da ermittelt wurde. Der N-Terminus und die ersten 33 Aminosäuren wurden über Edman Abbau bestimmt (Applied Biosystems Gasphasen-Sequencer 473 A). Aus diesen Daten leiten sich die Aminosäuresequenz von COM mit 134 Aminosäuren und ein theoretisches Molekulargewicht von 15566 Da ab, dabei wird berücksichtigt, dass die sechs Cysteinreste drei Disulfidbrücken ausbilden. Die bestimmte Aminosäuresequenz von COM lautet:

1 ELTEAQRRGL QVALEEFHKH PPVQWAFQET SVESAVDTPF PAGIFVRLEF
51 KLQQTSCRKR DWKKPECKVR PNGRKRKCLA CIKLGSEDKV LGRLVHCPIE
101 TQVLREAEEH QETQCLRVQR AGEDPHSFYF PGQF

### Beispiel 6: Klonierung und rekombinante Expression von TIG2 in CHO Zellen

Die cDNA für humanes TIG2 (Genbank Accession No. U77594) wurde aus Leber cDNA über PCR mit den Primern 5' GCCAGGGTGACACGGAAG 3' (TIG2oli1) und 5' GAGGCACCACGCAGCTC 3' (TIG2oli2) amplifiziert. Dabei wurde ein Fragment von 537 bp erhalten, das in den Vektor pGEM5Zf-T oder andere übliche Vektoren subkloniert wurde. Die Sequenz wurde über DNA-Sequenzanalyse überprüft und bestätigt. Aus diesem rekombinanten Vektor wurde ein Fragment, welches die cDNA von TIG2 enthält, mittels geeigneter Restriktionsenzyme ausgeschnitten, und in den Expressionvektor pCI oder andere übliche Expressionsvektoren subkloniert. CHO Zellen wurden mit dem rekombinanten Expressionsvektor wie unter Beipiel 2 transfiziert und stabile Zellklone wie unter Beispiel 2 selektioniert. Die erhaltenen Zellklone wurden auf Expression von TIG2 über RT-PCR untersucht.

### Beispiel 7: Aktivierung von GORI-28 mit rekombinantem TIG2

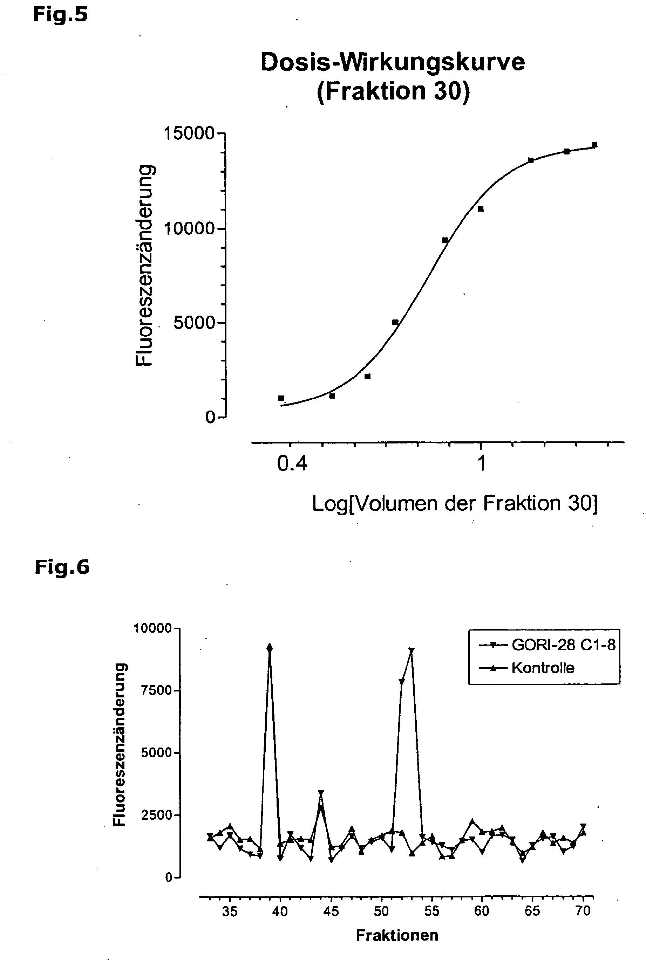

Ein TIG2-exprimierender Zellklon wurde für die Produktion von TIG2 expandiert. Vier konfluente 75 cm Flaschen wurden zweimal mit PBS gewaschen und anschliessend Medium ohne FKS appliziert. Nach 72 h Inkubation wurde das konditionierte Medium abgenommen, 5 min mit 500 g zentrifugiert, um Zelltrümmer zu entfernen, und anschliessend über eine Source RPC15 (10 x 250 mm) aufgereinigt. Die erhaltenen Fraktionen wurden im FLIPR Assay (s. Beispiel 3) auf GORI-28-stimulierende Aktivität getestet. In den Fraktionen 52 und 53 wurde Aktivität gefunden, die GORI-28 Zellen nicht aber eine Kontrollzelllinie stimulieren (s. Fig. 6). Zur Kontrolle wurde konditioniertes Medium von CHO Zellen verwendet, die ein anderes Peptid exprimieren. In diesem konditionierten Medium wurde keine GORI-28 stimulierende Aktivität gefunden (nicht dargestellt).

### Beispiel 8: Klonierung und rekombinante Expression von COM in Hefe

Über PCR mit den Primern 5' CTCTCGAGAAAAGAGAGCTCACGGAAGCCCAGC 3' (COMoli1) und 5' TTGTCGACTTAGAACTGTCCAGGGAAGTAGAAGC 3' (COMoli2) wurde unter Verwendung der in Beipiel 6 klonierten TIG2 cDNA ein 427 bp Fragment amplifiziert, welches die cDNA für COM darstellt, und anschließend in den Vektor pGEM5Zf-T oder andere übliche Vektoren subkloniert. Nach Bestätigung der Sequenz über DNA-Sequenzanalyse wurde aus dem rekombinanten Vektor mittels geeigneter Restriktionsenzyme ein Fragment ausgeschnitten und in den modifizierten Hefe-Expressionsvektor YEpFLAG-1 oder andere übliche Expressionsvektoren subkloniert. Über Elektroporation wurde das Expressionskonstrukt in den Hefestamm BJ3505 oder andere übliche Hefestämme transformiert. Die dabei entstehenden ADH2+-Klone wurden über PCR-Analyse auf Insertion der COM-DNA ins Hefegenom überprüft. Zur Herstellung von rekombinanten COM wurden 10 ml Kulturen mit COM-positiven Klonen angeimpft und die Expression induziert. Nach 96 h wurden die Zellüberstände geerntet und auf die Expression des rekombinanten COM nach Auftrennung über ein Gel (SDS-PAGE) und Anfärbung mit Coomassieblau überprüft. In dem beschriebenen Hefeexpressionssystem wird die cDNA von COM an das N-terminale Signal des Hefe Alpha-Faktors fusioniert.

Die Alpha-Faktor Signalsequenz bewirkt, dass das Fusionsprodukt ins Zellmedium sezerniert wird. Die Alpha-Faktor Signalsequenz wird durch die endogene Protease Kex 2 abgespalten und dabei entsteht reifes COM.

### Beispiel 9: Aufreinigung von rekombinantem COM und Nachweis der Aktivität

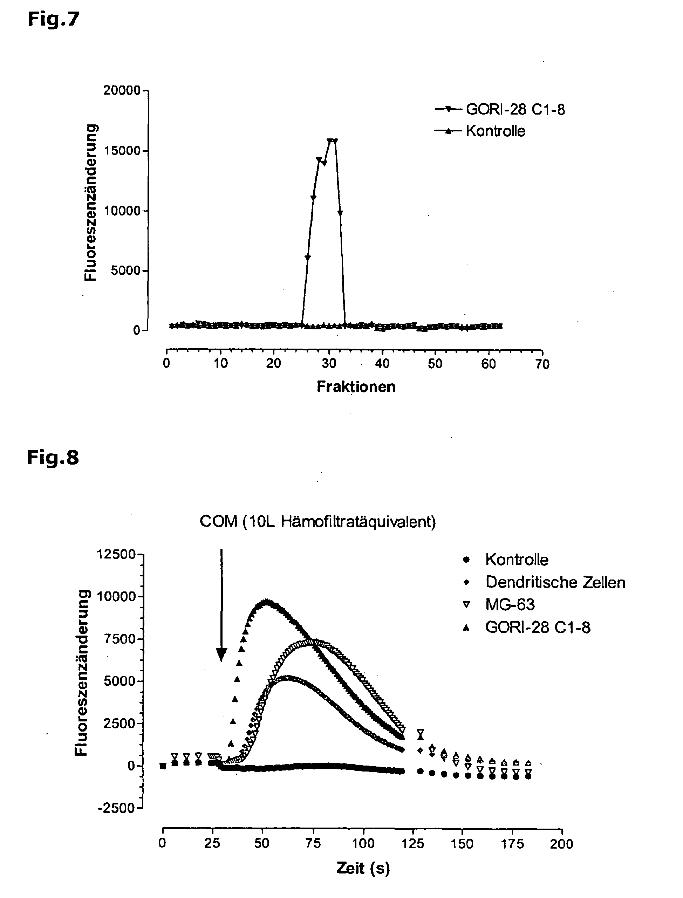

Von einem COM-exprimierenden Hefeklon wurde aus dem Zellüberstand rekombinantes COM aufgereinigt. Der Zellüberstand wurde filtriert (0,2 µM Filter), mit Puffer A (10 mM Na2HPO4, pH 7.0) 1/3 verdünnt, auf eine Heparinsäule (Hightrap) aufgetragen und mit Puffer B (Puffer A mit 1.5 M NaCl) eluiert. Die erhaltenen Fraktionen wurden im FLIPR Assay (s. Beispiel 3) zur Lokalisierung der GORI-28-stimulierenden Aktivität getestet. Im zweiten Aufreinigungsschritt wurden die aktiven Fraktionen vereinigt, auf eine RPC15 Säule aufgetragen und eluiert. Die aktiven Fraktionen wurden wie zuvor über den funktionellen Assay bestimmt, vereinigt und im dritten Aufreinigungsschritt über eine Phenomenex C18 Säule aufgereinigt. Aufgereinigtes rekombinantes COM zeigt spezifische Aktivität auf GORI-28 Zellen, nicht aber auf einer Kontrollzelllinie, die den Rezeptor nicht exprimiert (s. Fig. 7).

### Beispiel 10 Aktivierung von osteogenen Zellen durch COM

Zur Untersuchung der funktionellen Aktivierung von Knochenzellen durch COM wurden MG-63 Zellen, eine etablierte humane osteosarkoma Zelllinie (Osteoblast-ähnlicher Typ), wie in Beispiel 3 beschrieben am Vortag mit 20.000 Zellen pro Loch in 96-Lochplatten ausgesät und im FLIPR-Assay getestet. Die Zellen wurden mit aus HF-gereinigtem COM (siehe Beispiel 4 und 5) stimuliert und zeigten eine COM-induzierte Freisetzung von Ca2+-Ionen (s. Fig. 8). Als Positiv- bzw. Negativkontrolle sind die GORI-28 überexprimierende CHO-Zelllinie und eine CHO-Zellinie, die einen anderen G-Protein gekoppelten Rezeptor exprimiert, gezeigt.

### Beispiel 11: Aktivierung von dendritischen Zellen durch COM

Dendritische Zellen wurden aus humanem Vollblut gewonnen. Zunächst wurden aus Vollblut (500 ml) über mehrere Zentrifugationsschritte und Separation mit Hilfe von paramagnetischen Antikörpern (Anti-CD14) monocytäre Zellen isoliert (CD14+). Die erhaltenen Vorläuferzellen wurden mit GM-CSF (800 U/ml) und Interleukin-4 (500 U/ml) behandelt, um die Differenzierung zu dendritischen Zellen zu induzieren (Inkubationszeit 7 Tage). Anschließend wurden die Zellen mit LPS stimuliert, um sogenannte reife dendritische Zellen zu erhalten. Diese reifen dendritischen Zellen wurden am Vortag in 96-Lochplatten mit einer Zelldichte von 20,000 Zellen/Loch ausgesät und anschließend, wie im Beispiel 3 beschrieben, im FLIPR-Assay getestet. Die Zellen wurden mit aus HF-gereinigtem COM (siehe Beispiel 4 und 5) stimuliert und zeigten eine COM-induzierte Freisetzung von Ca2+-Ionen (s. Fig. 8).

### Beispiel 12: Expression von TIG2 und GORI-28 in ausgewählten Zelllinien

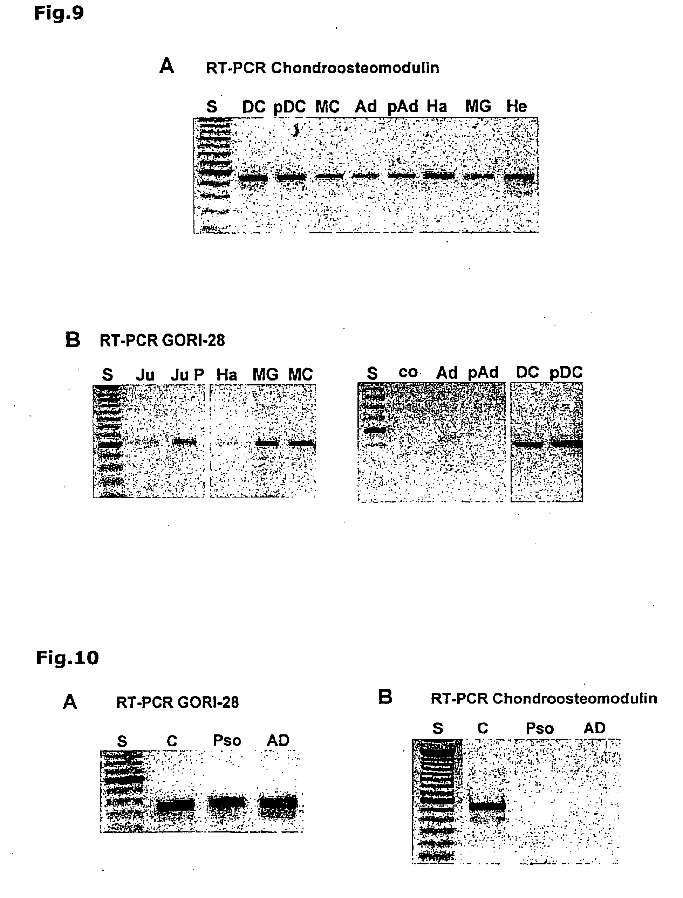

Die native Expression von TIG2 und GORI-28 in verschiedenen Zelllinien wurde über RT-PCR mit genspezifischen Primern untersucht. COM wurde mit dem Primerpaar TIG2oli1 und TIG2oli2 (s. Beispiel 6) als 537bp cDNA Fragment amplifiziert und gelelektrophoretisch dargestellt. In Analogie wurde der GORI-28 wurde mit Hilfe des Primerpaars 5' GGC CAT GTG CAA GAT CAG CAA CT 3' (mDEZoli1) und 5' AGA ATG GGG TTC ATG CAG CTG TT 3' (mDEZoli2) als 618 bp Fragment amplifiziert bzw. nachgewiesen; für die PCR Amplifikation aus murinen Adipozyten wurde das Primerpaar 5' TCT ACA ACG GTG GAA CAG TGA 3' (mDEZoli3) und 5' AAG AAA GCC AGG ACC CAG A 3' (mDEZoli4) eingesetzt, dabei entsteht ein 536 bp Fragment; für die Amplifikation aus humanen dendritischen Zellen wurde das Primerpaar 5' CAG ACA ACA TAA CGG TGA ATG A 3' (hDEZ_a_Oli5) und 5' AAG AAA GCC AGG ACC CAG A 3' (hDEZ_a_Oli4) eingesetzt, dabei entsteht ein 524 bp Fragment. Von den zu untersuchenden Zellen wurden aus Zellproben nach üblichen Methoden RNA isoliert, die dann in eine Erststrang cDNA umgeschrieben wurde und für die PCR-Amplifikation eingesetzt wurde.
Die Expression des Liganden COM konnte in reifen humanen dendritischen Zellen (DC) sowie Vorläuferzellen (pDC), murinen Osteosarkomzellen MC3T3 (Osteoblast-ähnlicher Typ, MC), reifen murinen Adipozyten (Fettzellen, Ad) sowie Vorläuferadipozyten (pAd), humanen Keratinozyten (HaCaT, Ha), humanen Osteosarkomzellen MG-63 (Osteoblast-ähnlicher Typ, MG) und in humanen Hepatozyten HepG2 (He) detektiert werden (s. Fig. 9 A).

Die Expression des COM-Rezeptors GORI-28 wurde in Jurkat T-Zellen (humane leukämische T-Zelllinie, Ju), verstärkt in PMA/Ionomycin aktivierten Jurkat T-Zellen (Ju P), in HaCaT-Zellen (Ha), MG-63 Zellen (MG), MC3T3 (MC), dendritischen (DC) sowie Vorläuferzellen (pDC), in reifen Adipozyten (Ad) nicht aber in Vorläuferzellen (pAd) nachgewiesen (s. Fig. 9 B). In Fig. 9B ist eine Negativkontrolle (co) gezeigt, in der die cDNA als Template gegen eine Wasserprobe ersetzt wurde.
Die Expressionsanalyse des COM Rezeptors GORI-28 zeigt, dass seine Expression durch physiologische Prozesse gesteuert wird. Im dargestellten Beispiel konnte die Expression des Rezeptors durch in vitro T-Zell Aktivierung erhöht und in unreifen Adipozyten durch Differenzierung induziert werden. Die T-Zell Aktivierung wurde in Jurkat T-Zellen mittels Phorbolester und Ionomycin erzielt. Die Differenzierung der unreifen Adipozyten wurde durch Dexamethason, 8BrcAMP und Insulin induziert.

### Beispiel 13: Expression von COM und GORI-28 in Hautzellen von Patienten mit Hauterkrankungen

Wie in Beipiel 12 beschrieben wurde die Expression von COM und seinem Rezeptor GORI-28 über RT-PCR ermittelt. Für die Amplifikation des GORI-28 wurde in diesem Fall das Primerpaar 5' GCA CAG CAT CAC TTC TAC CAC TT 3' (hDEZoli3) und 5' CTG TAG ACC ACC ACC AGG AAG A 3' (hDEZoli2) verwendet, dabei entsteht ein 324 bp Fragment. Hautstanzen von Patienten ohne Hauterkrankung (Kontrolle, C) und von Patienten die unter Psoriasis (Pso) oder atopischer Dermatitis (AD) leiden wurden über eine Hautklinik bezogen. Das Material wurde nach üblichen Methoden aufbereitet, um RNA zu isolieren, von der wiederum wie in Beispiel 12 beschrieben eine Erststrang cDNA synthetisiert wurde, die für die PCR-Amplifikation eingesetzt wurde. Der Rezeptor GORI-28 wird sowohl im Hautgewebe von Gesunden wie auch im Hautgewebe von Patienten mit Psoriasis oder atopischer Dermatitits exprimiert (s. Fig. 10 A). Im Gegensatz dazu konnte COM-Expression nur im Gewebe von Gesunden, nicht aber im Hautgewebe von Patienten die unter Psoriasis oder atopischer Dermatitits leiden nachgewiesen werden (s. Fig. 10 B). Damit steht die fehlende Expression von COM im kausalen Zusammenhang von Hauterkrankungen und weist auf eine therapeutische Wirksamkeit von COM für dieses Indikationsgebiet hin.

Zusammenfassend konnte gezeigt werden, dass im menschlichen Blutfiltrat die zirkulierende Substanz COM vorkommt, die isoliert und charakterisiert werden konnte. Sie übt ein osteochondroanabole, immunmodulatorische, sowie Haut- und Energiestoffwechsel- regulierende Wirkungen aus.

## Patentansprüche

1. COM mit der Aminosäuresequenz SEQ ID Nr. 1: 
1 ELTEAQRRGL QVALEEFHKH PPVQWAFQET SVESAVDTPF PAGIFVRLEF
51 KLQQTSCRKR DWKKPECKVR PNGRKRKCLA CIKLGSEDKV LGRLVHCPIE
101 TQVLREAEEH QETQCLRVQR AGEDPHSFYF PGQF
 sowie amidierte, acetylierte, phosphorylierte und glycosylierte Derivate oder mit einem Pyroglutamat am N-Terminus,
wobei
die Aminosäuresequenz der Derivate des COM durch Aminosäureaustausche, -insertionen oder -deletionen verändert ist, mit den Maßgaben, dass
- die Derivate eine Länge von 129-140 Aminosäuren aufweisen,
- die Derivate zu COM zu mehr als 80% Sequenzidentität aufweisen,
- die Derivate im funktionellen Test mit dem FLIPR-System den Rezeptor GORI-28 aktivieren, so dass eine Rezeptoraktivität gemessen wird, die mindestens 80% der unter gleichen Testbedingungen von COM ausgelösten Rezeptoraktivität beträgt, vorzugsweise jedoch über der durch COM ausgelösten Rezeptoraktivität liegt.

2. COM oder ein Derivat nach Anspruch 1 und dessen Rezeptor GORI-28 als Ligand-Rezeptor-System.

3. Verwendung eines Ligand-Rezeptorsystems nach Anspruch 2 zum Screening von Substanzen aus Peptidbibliotheken oder anderer Stoffbanken und als Drug Target.

4. Verfahren zur Herstellung von COM oder seiner Derivate nach Anspruch 1, **dadurch gekennzeichnet, dass** es über Zellkulturen hergestellt und chromatographisch gereinigt wird, oder dass es aus menschlichem Blut über übliche Chromatographie-Verfahren in bekannter Weise isoliert wird, oder dass es durch die üblichen Verfahren der chemischen oder biotechnologischen Synthese hergestellt und mit den gängigen Chromatographie-Verfahren gereinigt wird.

5. Arzneimittel enthaltend COM oder seine Derivate nach Anspruch 1, gegebenenfalls neben üblichen Hilfs- und Zusatzstoffen.

6. Arzneimittel nach Anspruch 5, insbesondere als Lyophilisat, wobei die galenische Applikationsform mit Mannit aufgenommene Form in sterilen Ampullen zur Auflösung in physiologischer Kochsalzlösung und/oder Infusionslösungen zur wiederholten Einzelinjektion und/oder Dauerinfusion in Mengen von 300 Mikrogramm bis 30 Milligramm reines COM pro Therapie-Einheit enthält.

7. Verwendung von COM sowie seiner Derivate nach Anspruch 1 zur Herstellung eines Arzneimittels zur Behandlung von Erkrankungen der Nebenschilddrüse, insbesondere bei deren Unterfunktion (Hypoparathyreodismus),
- zur Behandlung von degenerativen Knochenerkrankungen, insbesondere der Osteoporose,
- zur Behandlung von Knochenfrakturen in der Heilungsphase,
- zur Behandlung von Knorpelerkrankungen, Bindegewebserkrankungen, Rheuma und Arthrose,
- zur Behandlung von Fettsucht und Diabetes Typ 2,
- zur Behandlung von Nierenerkrankungen, die mit Störungen der Elektrolytausscheidung einhergehen, insbesondere bei der akuten Niereninsuffizienz sowie Phosphat- und Calciumausscheidungsstörungen, oder zur Behandlung von Hauterkrankungen, insbesondere Psoriasis, Ekzemen und Akne.

8. Verwendung von COM sowie seiner Derivate nach Anspruch 1 zur Herstellung eines Diagnostikmittels zur Diagnose von Erkrankungen, nach Anspruch 7, wobei spezifische Antikörper gegen das synthetische Molekül hergestellt werden und die Blutkonzentration von COM über Immuntests oder über quantitative Massenspektrometrie gemessen wird.

9. Verwendung von COM sowie seiner Derivate zur Herstellung eines Arzneimittels nach Anspruch 7 in verschiedenen galenischen Applikationsformen, insbesondere als Lyophilisat.

10. Verwendung nach Anspruch 9, wobei die galenische Applikationsform, insbesondere die lyophilisierte, mit Mannit aufgenommene Form in sterilen Ampullen zur Auflösung in physiologischer Kochsalzlösung und/oder Infusionslösungen zur wiederholten Einzelinjektion und/oder Dauerinfusion in Mengen von 300 Mikrogramm bis 30 Milligramm reines COM pro Therapie-Einheit enthält.

## Claims

1. COM having the amino acid sequence SEQ ID No. 1 
1 ELTEAQRRGL QVALEEFHKH PPVQWAFQET SVESAVDTPF PAGIFVRLEF
51 KLQQTSCRKR DWKKPECKVR PNGRKRKCLA CIKLGSEDKV LGRLVHCPIE
101 TQVLREAEEH QETQCLRVQR AGEDPHSFYF PGQF
 and amidated, acetylated, phosphorylated and glycosylated derivatives thereof, or having a pyroglutamate at the N terminus, in which the amino acid sequence of the derivatives of COM is changed by amino acid substitutions, insertions or deletions, with the provisos that
- the derivatives have a length of from 129 to 140 amino acids;
- the derivatives have a sequence identity with COM of more than 80%;
- the derivatives will activate the receptor GORI-28 in a functional test with the FLIPR system, so that a receptor activity is measured which is at least 80% of the receptor activity triggered by COM under the same testing conditions, preferably being greater than the receptor activity triggered by COM.

2. The COM or derivative according to claim 1 and its receptor GORI-28 as a ligand-receptor system.

3. Use of a ligand-receptor system according to claim 2 for the screening of substances in peptide libraries or other substance libraries and as a drug target.

4. A method for the preparation of COM or its derivatives according to claim 1, **characterized in that** it is prepared by cell cultures and purified by chromatography, or that it is isolated from human blood through usual chromatographic methods in the usual way, or that it is prepared by the usual methods of chemical or biotechnological synthesis and purified by the usual chromatographic methods.

5. A medicament containing COM or its derivatives according to claim 1, optionally in addition to usual adjuvants and additives.

6. The medicament according to claim 5, especially a lyophilized form taken up with mannitol, wherein the galenic dosage form contains amounts of from 300 µg to 30 mg of pure COM per therapy unit in sterile ampoules for dissolution in physiological saline and/or infusion solutions for the repeated single injection and/or permanent infusion.

7. Use of COM and its derivatives according to claim 1 for the preparation of a medicament for the treatment of diseases of the parathyroid gland, especially its hypofunction (hypoparathyroidism);
for the treatment of degenerative bone diseases, especially osteoporosis;
for the treatment of bone fractures in the phase of healing;
for the treatment of cartilage diseases, connective tissue diseases, rheumatism and arthrosis;
for the treatment of obesity and diabetes type 2;
for the treatment of renal diseases accompanied by disorders in electrolyte excretion, especially in acute renal insufficiency and phosphate and calcium excretion disorders; or
for the treatment of skin diseases, especially psoriasis, eczemas and acne.

8. Use of COM and its derivatives according to claim 1 for the preparation of a diagnostic agent for the diagnosis of diseases according to claim 7, wherein specific antibodies against the synthetic molecule are prepared and the blood concentration of COM is measured by immune tests or by quantitative mass spectrometry.

9. Use of COM and its derivatives for preparing a medicament according to claim 7 in various galenic dosage forms, especially as a lyophilizate.

10. The use according to claim 9, wherein said galenic dosage form, especially the lyophilized form taken up with mannitol, contains amounts of from 300 µg to 30 mg of pure COM per therapy unit in sterile ampoules for dissolution in physiological saline and/or infusion solutions for the repeated single injection and/or permanent infusion.

## Revendications

1. COM ayant la sequence d'acides amines SEQ ID N° 1 : 
1 ELTEAQRRGL QVALEEFHKH PPVQWAFQET SVESAVDTPF PAGIFVRLEF
51 KLQQTSCRKR DWKKPECKVR PNGRKRKCLA CIKLGSEDKV LGRLVHCPIE
101 TQVLREAEEH QETQCLRVQR AGEDPHSFYF PGQF
 et derives amides, acetyles, phosphoryles et glycosyles ou avec un pyroglutamate au niveau de l'extremite N-terminale, dans laquelle
la sequence d'acides amines des derives de COM a ete modifiee par substitutions, insertions ou deletions d'acides amines, à condition que
- les derives aient une longueur de 129 à 140 acides amines,
- les derives presentent une identite de sequence avec COM superieure à 80%,
- les derives, dans le cadre d'un essai fonctionnel avec le syst'me FLIPR, activent le recepteur GORI-28 de sorte qu'une activite receptrice, qui correspond à au moins 80% de l'activite receptrice provoquee par COM dans les mêmes conditions d'essai, mais qui est de preference superieure à l'activite receptrice provoquee par COM, est mesuree.

2. COM ou derive selon la revendication 1 et son recepteur GORI-28 en tant que syst'me ligand-recepteur.

3. Utilisation d'un syst'me ligand-recepteur selon la revendication 2 pour cribler des substances dans des banques de peptides ou d'autres banques de substances et en tant que cible de medicament.

4. Procede de preparation de COM ou de ses derives selon la revendication 1, **caracterise en ce qu**'elle est produite en utilisant des cultures de cellules et purifiee par chromatographie, ou en ce qu'elle est isolee de manière connue à partir de sang humain au moyen de procedes chromatographiques classiques, ou en ce qu'elle est produite au moyen des procedes classiques de synth'se chimique ou biotechnologique et purifiee au moyen de procedes chromatographiques habituels.

5. Medicament contenant COM ou ses derives selon la revendication 1, eventuellement avec les additifs et adjuvants habituels.

6. Medicament selon la revendication 5, en particulier sous forme de lyophilisat, dans lequel la forme galenique contient la forme absorbee dans du mannitol dans des ampoules steriles pour une dissolution dans du serum physiologique et/ou des solutions pour perfusion destinees à des injections individuelles repetees et/ou une perfusion continue à raison de 300 microgrammes à 30 milligrammes de COM pure par unite therapeutique.

7. Utilisation de COM et des ses derives selon la revendication 1 pour la preparation d'un medicament destine au traitement de maladies de la glande parathyroïde, en particulier dans le cas d'un hypofonctionnement (hypoparathyroïdie),
- destine au traitement de maladies osseuses degeneratives, en particulier de l'osteoporose,
- destine au traitement de fractures osseuses en phase de consolidation,
- destine au traitement de maladies cartilagineuses, de maladies du tissu conjonctif, des rhumatismes et de l'arthrose,
- destine au traitement de l'obesite et du diab'te de type 2,
- destine au traitement de maladies renales qui s'accompagnent de troubles de l'excretion d'electrolytes, en particulier en cas d'insuffisance renale aiguë, ainsi que de troubles de l'excretion du phosphate et du calcium, ou
- destine au traitement de maladies de la peau, en particulier du psoriasis, de l'eczema et de l'acne.

8. Utilisation de COM et de ses derives selon la revendication 1 pour la preparation d'un agent diagnostique destine au diagnostic de maladies selon la revendication 7, dans laquelle des anticorps specifiques diriges contre la molecule synthetique sont produits et la concentration sanguine de COM est mesuree au moyen d'un dosage immunologique ou d'une spectrometrie de masse quantitative.

9. Utilisation de COM et de ses derives pour la preparation d'un medicament selon la revendication 7 dans diverses formes galeniques, en particulier sous forme de lyophilisat.

10. Utilisation selon la revendication 9, dans laquelle la forme galenique contient en particulier la forme lyophilisee absorbee dans du mannitol dans des ampoules steriles pour une dissolution dans du serum physiologique et/ou des solutions pour perfusion destinees à des injections individuelles repetees et/ou une perfusion continue à raison de 300 microgrammes à 30 milligrammes de COM pure par unite therapeutique.
